# NEW EUROPEAN PATENT SPECIFICATION

(11) **EP 4 157 165 B2**
(45) Date of publication and mention of the opposition decision: **22.07.2026**
(45) Mention of the grant of the patent: 09.08.2023
(21) Application number: 21734458.9
(22) Date of filing: 01.06.2021
(51) Int. Cl.: A61F 5/44, A61F 5/443, A61F 5/441, A61F 5/445

(54) **AN OSTOMY POUCH**
OSTOMIEBEUTEL
POCHE DE STOMIE

(30) Priority: 02.06.2020 GB 202008261
(43) Date of publication of application: 05.04.2023
(73) Proprietor: ConvaTec Limited, Deeside, Flintshire CH5 2NU (GB)
(72) Inventor: EVANS, Kevin, Deeside, Flintshire CH5 2NU (GB)
(74) Representative: D Young & Co LLP
(86) International application number: PCT/GB2021/051339
(87) International publication number: WO 2021/245396

(56) References cited:
- WO-A1-2013/142577
- CN-U- 208 989 319
- DE-A1- 102014 104 737
- US-A- 4 411 659
- US-B2- 7 559 922
- SALTSHEALTHCARE: "How does our Active Chamber Filter System work? | Salts Healthcare", YOUTUBE, XP093190517, Retrieved from the Internet <URL:https://www.youtube.com/watch?v=MkNTbXEDjPA> [retrieved on 20240507]

## Description

### Technical Field of the Invention

The present invention relates to an ostomy pouch having a cavity for containing a stomal output and a filter system for venting gas from the pouch. The invention is defined in the claims.

### Background to the Invention

An ostomy pouch may be used to collect and hold stomal output from a stoma formed in the body of an ostomate. Generally the stoma is a surgical opening in the torso of the ostomate's body, but may also refer to internal tissue, organs or portions thereof that are exposed by the opening. Ostomy pouches typically take the form of a pair of walls sealed together to form a cavity into which stomal output may be expelled from the ostomate through a formed stoma.

Given the nature and use of ostomy pouches, it is desirable for a pouch to be able to be worn by an ostomate as easily and discreetly as possible. It is also advantageous for the pouch to be worn for an extended period of time for increased convenience for the ostomate. However, in order to increase the length of time a pouch can be used it is typically necessary to increase the volume of the pouch, which may be detrimental in terms of the discreetness of the pouch, in use. Some ostomy pouches may be provided with a drain allowing the ostomate to drain stomal output from the cavity. This may give the ostomate added control over the amount of output within the cavity at any one time, but at the cost of reduced convenience where the ostomate is required to intermittently drain the pouch.

Further, gaseous stomal output may accumulate in the ostomy pouch, which may result in the ostomy pouch expanding unless the gas is vented from the pouch. The associated expansion may increase the likelihood of the ostomy pouch moving from its original position during use. In addition to forming a visible bulge under the ostomate's clothing, any movement of the ostomy pouch may result in leaking or the ostomy pouch falling off the ostomate. Therefore, some ostomy pouches include an outlet for venting the gaseous stomal output from the pouch.

Where an outlet is provided, it is desirable to prevent liquid and/or solid stomal output from coming into contact with the outlet, so as to prevent the outlet from becoming blocked or clogged, or even preventing leakage of liquid and/or solid output through the outlet. However, to date, no complete solution has been provided which address these fallbacks.

WO 2013/142577 A1 discloses an ostomy pouch comprising a first potion and a second portion. The potions are configured such that the second portion can be inverted and inserted within the first portion in a compacted state. An inner surface of an outer wall of the first portion comprises a filter and the inverted second portion can be arranged between an inlet opening and the filter in the compacted state.

US 7559922 B2 discloses a filter system comprising a vent and a pre-filter arrangement comprising a wall which defines a filter chamber. The filter chamber comprises an opening allowing gaseous stomal output to migrate into the filter, and a microporous membrane that is hydrophobic and oleophobic. The pre-filter arrangement is configured to control the content of stomal output inContact with the vent.

CN 208989319 U discloses an ostomy bag with a filtering system. He ostomy bag comprises a bag body with a front film and a rear film, a rubber disc arranged on the front film and a filtering system arranged in the bag body. The filtering system comprises a protective layer covering the top of the inner wall of the rear film, a first filtering piece arranged on the protective layer and a second filtering piece arranged on the top of the rear film.

US 4411659 A discloses an ostomy bag comprising a gas venting and filtering assembly having a replaceable deodorizing filter element.

It is therefore an aim of an embodiment or embodiments of the invention to overcome or at least partially mitigate one or more problems with the prior art.

### Summary of the Invention

According to an aspect of the invention there is provided an ostomy pouch according to claim 1.

Advantageously, the ostomy pouch of the present invention provides improved management of stomal output within the cavity compared with prior art solutions. Specifically, the present ostomy pouch allows for the various gaseous, liquid and solid components of the output to be stored, or vented as required whilst maintaining convenience and discreetness for the user. This is achieved by providing both an increased cavity volume for the pouch which, in conjunction with the waisted section controls (i.e. prevent or substantially reduce) sagging of the pouch when filled with stomal output, allowing for a greater volume of stomal output to be contained within the pouch before an ostomate feels it necessary to empty or replace the pouch, and means for venting gaseous stomal output from the cavity, preventing "bloating" or "ballooning" of the pouch. The pre-filter arrangement of the present invention provides additional control over the separation of solid, liquid and gaseous components of the stomal output, ensuring continued operation of the vent.

Optional features set out below may apply to any aspect of the invention as appropriate.

The pre-filter arrangement may be configured to control the content of stomal output in contact with the vent. For example, the pre-filter may be advantageously configured to prevent or at least reduce the level of solid or liquid stomal output able to come into contact with the vent.

The filter chamber may comprise an inlet allowing gaseous stomal output to migrate into the filter chamber.

The inlet may comprise one or more apertures in the protective panel.

In embodiments, the inlet comprises a plurality of apertures in the protective panel. The one or more apertures may comprise a cut or slot in the protective panel. The one or more apertures may comprise a c-cut or an s-cut in the protective panel. In embodiments, the inlet comprises a plurality of c-cuts in the protective panel.

The one or more apertures may be positioned about the vent. For example, the one or more apertures may be positioned at a radial distance from the vent. The one or more apertures may be positioned at a radial distance of between 10mm to 40mm, or between 15mm to 35mm, or between 20mm to 30mm, or between 23mm to 27mm, for example.

The one or more apertures may be angled with respect to a horizontal axis through the pouch, with the pouch in its in-use orientation. For example, in some embodiments the inlet may comprise a plurality of c-cuts in the protective panel, each angled at approximately 45° with respect to a horizontal axis through the pouch.

The inlet may be located above the vent with the pouch in its in-use orientation.

The outlet is provided to prevent liquid stomal output being in contact with the vent for a prolonged period of time. For example, when the ostomate moves around with the ostomy pouch attached they may change the orientation of the ostomy appliance such that the stomal liquid is held by gravity in contact with the filter chamber inlet (e.g. if the ostomy pouch is upside down or on its side when the ostomate is lying down). Under such conditions, some stomal liquid may enter the filter chamber via the inlet. Beneficially, when the ostomate returns to standing or sitting upright the outlet allows any stomal liquid that has entered the filter chamber to exit.

In embodiments, the inlet for the filter chamber may also act as the outlet for liquid stomal output. In such instances, the inlet/outlet is provided below the vent with the pouch in its in-use orientation.

In some embodiments the outlet is formed between the protective panel and a wall of the pouch.

The outlet may be formed between the protective panel and the outer wall of the pouch. The protective panel may be attached to a wall of the pouch at one or more attachment regions. It may be attached to the inner wall, or more preferably to the outer wall of the pouch. The one or more attachment regions may thereby define an outlet in the form of one or more gaps between adjacent attachment regions, and/or between an attachment region and the peripheral seal forming the cavity.

The protective panel may be attached to the inner wall, but preferably the outer wall of the pouch at the one or more attachment regions by one or more welds. The one or more welds may comprise one or more, preferably a plurality, of spot welds. In embodiments, the plurality of spot welds define multiple attachment regions and multiple gaps therebetween. In other embodiments, the one or more welds comprise one or more, preferably a pair of, bar welds. In embodiments, the pair of bar welds define a pair of attachment regions with a single gap therebetween. Alternatively, the one or more welds may comprise a single bar weld defining a single attachment region spanning substantially the entire width of the pouch, with a single gap provided between an end of the attachment region and the peripheral seal forming the cavity.

The protective panel may be attached to the inner and outer walls about part of the periphery thereof, for example along one or more sides and/or the uppermost edge of the protective panel. The attachment may be provided in the outline weld between the inner and outer walls of the pouch. The protective panel may be attached to the inner and outer walls about its periphery except for at least a portion of a lowermost edge of the protective panel where it may be attached to the inner but preferably outer wall at the one or more attachment regions, only.

The outlet may be located below the vent with the pouch in its in-use orientation. Where separate inlets and outlets are present, the outlet may be located below the inlet with the pouch in its in-use orientation.

The filter system may comprise an odour filter. The odour filter may be provided at or proximal to the vent. The odour filter may comprise a charcoal or activated carbon filter, for example. The odour filter may be substantially circular or disc shaped. A major face of the circular/disc shaped filter may be permeable allowing for gaseous stomal output to enter the filter therethrough. The odour filter may comprise a strip filter which may have open ends, for example.

The filter system, or components thereof - e.g. the vent - may be provided within or be associated with the outer wall of the ostomy pouch. For example, in some embodiments, the filter system may comprise an odour filter positioned on an exterior surface of the outer wall of the pouch. In such embodiments, the vent may comprise an opening within the outer wall, e.g. a substantially circular opening, positioned proximal to the odour filter and providing a vent through which gaseous stomal output may exit the interior of the pouch and enter the odour filter, in use. Advantageously, having the odour filter exterior to the interior of the pouch may minimise exposure of the filter to stomal output, particularly solid and liquid stomal output which may clog the filter if exposed for an extended period of time, and in doing so may result the pouch to undesirably bloat or balloon. Such an arrangement may be particularly useful for open pouches, for example, where the same pouch may be used for a prolonged period by a user.

In other embodiments the filter system may comprise an odour filter provided on an interior surface of the outer wall of the pouch. In such embodiments, the vent may comprise one or more slits, openings or the like within the outer wall of the ostomy pouch. The slits may be positioned adjacent to the odour filter, for example adjacent to a rear face of the odour filter which is adhered or otherwise coupled to the outer wall of the pouch. Whilst suffering the drawbacks of the potential of having the liquid and solid stomal output in contact with the odour filter for extended periods, embodiments wherein the odour filter is provided within the interior of the pouch may provide a cost-effective solution, and may be particularly suited for use with closed pouches, for instance, which are designed for wear for a much shorter period of time when compared with open pouches. Here, the filter may be exposed to the stomal output, but for a much shorter period of time and therefore may be less likely to clog before the end of its use period.

The filter system may be provided within, or be associated with the upper section of the pouch.

The filter system may be provided with a filter cap. The filter cap may be provided on an exterior surface of the outer wall of the pouch, or an exterior surface of an outer comfort layer, where present, for example, and may be positioned about the an odour filter forming part of the filter system of the pouch. The filter cap may provide protection for the filter system, and in particular may be provided about and be operable to protect an odour filter, in use. The filter cap may include one or more openings or slits therein, e.g. one or more s-slits, which allow for the venting of gas therethrough.

The filter system may additionally include a filter cover label. The filter cover label may comprise a removeable component which may be positioned over the filter cap, in use, to seal the openings/slits therein. This may be particularly useful, for example, where an ostomate plans to swim, bathe or shower. The label may prevent ingress of water through said openings/slits in the cap and thereby prevent clogging of the odour filter from water.

The protective panel may be positioned within the cavity to define a filter chamber in the upper section of the pouch. For example, the protective panel may be attached to the inner or outer wall of the pouch at, proximal to, or above the waisted section, defining a filter chamber within the upper section of the pouch. Providing a protective panel which defines the filter chamber in the upper section of the pouch only may advantageously reduce the amount of material required to form the pouch and thereby providing benefits in terms of cost and manufacture. Alternatively, the protective panel may be positions within the cavity to define a filter chamber which spans the upper and lower sections of the pouch, or is located solely within the lower section of the pouch.

The cavity is sub-divided into two or more cavity chambers, separate from the filter chamber. The pouch comprises a separation wall between the inner and outer walls defining the cavity into first and second cavity chambers. The separation wall comprises a filtering element. The filtering element is fluid-permeable, and is operable to filter fluid stomal output from solid stomal output.

The filtering element may include an array of apertures allowing the passage of fluid stomal output therethrough. The apertures may have a diameter of between 0.02mm to 0.10mm, or between 0.03mm to 0.08mm, or between 0.04mm to 0.06mm, or between 0.06mm to 0.08mm, or between 0.10mm to 0.40mm, for example. The spacing between adjacent apertures in the array may be between 0.80mm to 2.20mm, or between 1.00mm to 2.00mm, or between 1.25mm to 1.75mm, for example. The apertures may extend across at least 50%, or at least 75%, or at least 80%, or at least 90% of the surface of the filtering element. The filtering element may extend across at least the lower half or lower quarter of the cavity, and/or may extend across at least the upper half or upper quarter of the cavity.

In use, the first cavity chamber is arranged to receive both fluid and solid stomal output from the ostomate via a stomal inlet in the inner wall of the pouch. The separation wall is arranged to allow passage of fluid stomal output into the second cavity chamber, retaining the solid stomal output in the first cavity chamber. In such embodiments, the filter system, and in particular the pre-filter is arranged to allow gaseous stomal output to pass into the filter chamber, retaining (as far as possible) fluid stomal output in the second cavity chamber, and allowing the gaseous stomal output to be removed from the cavity through the vent.

The pouch may further comprise a membrane positioned at least partly over the vent and/or odour filter (where present). The membrane may be a gas-permeable membrane. Advantageously, the gas-permeable membrane may prevent (or at least reduce the likelihood of) liquid and/or solid stomal output coming into contact with the vent and/or filter. The membrane may be provided with a coating thereon, e.g. a hydrophobic coating for reducing the likelihood of stomal output adhering to the membrane and reducing its performance.

The inner wall of the pouch may comprise an inlet for receiving the stomal output into the cavity. The inlet may be provided within a portion of the inner wall defining at least part of the upper section of the cavity.

The pouch may comprise a closed pouch. In alternative embodiments, the pouch may comprise an open pouch, and comprise a drain for releasing stomal output from the cavity. The drain may comprise a drain aperture comprising an opening within the pouch for releasing stomal output from the cavity. The drain and/or drain aperture may be defined, at least in part, by the inner and outer walls of the pouch.

The drain may comprise a deployable drain, which may be moveable between a stowed position and a deployed position. The deployable drain may be moveable between stowed and deployed positions by rolling or folding the drain, e.g. about one or more fold lines in the drain.

The pouch may comprise a fastening arrangement for retaining the drain in a stowed position. The fastening arrangement may comprise one or more fasteners. The one or more fasteners may comprise a pair of fastening elements. The fastening elements may comprise a strip of hook fasteners and a strip of loop fasteners together forming a hook and loop fastener arrangement. The fastening elements may comprise two strips of hook fasteners forming a dual hook fastener arrangement. The pouch may comprise a first fastening element located on the inner wall of the pouch. The second fastening element may be provided on the outer wall of the pouch, or in some embodiments on an outer comfort layer of the pouch. The second fastening element may be provided on a flap, which itself may be secured (e.g. adhesively or otherwise) to the outer wall or outer comfort layer (where present) of the pouch.

The drain may comprise one or more pursing strips. The pursing strip(s) may be associated with the inner or outer wall of the pouch. For example, the pursing strips may be adhesively or otherwise fixed to the inner or outer wall of the pouch. The pursing strip(s) may assist in separating the inner and outer walls of the pouch, in a portion thereof, to define a drain aperture in the pouch for draining stomal output from the cavity.

In embodiments, the drain is provided in the lower section of the pouch.

The maximum widths of the upper and lower sections may be equal (or substantially equal). The maximum width of the upper and lower sections may be different. For example, in some embodiments the maximum width of the upper section may be greater than the maximum width of the lower section.

The maximum width of the upper and/or lower section may be between 120mm to 170mm, or between 130mm to 160mm, or between 135mm to 150mm, or between 135 - 140mm, or between 140mm to 145mm, for example. In an exemplary embodiment, the maximum width of the upper section may be approximately 142mm, and the maximum width of the lower section may be between 137mm to 139mm.

The minimum width of the waisted section may be between 105mm to 135mm, or between 110mm to 130mm, or between 110mm to 125mm, or between 115mm to 130mm, or between 120mm to 135mm, or between 115mm to 120mm, or between 120mm to 125mm, or between 125mm to 130mm, or approximately 120mm, for example. In an exemplary embodiment, the minimum width of the waisted section may be approximately 129mm, 119mm or 109mm.

The minimum width of the waisted section may be between 5mm to 30mm less than the maximum width of the lower section, or between 10mm to 20mm less than the maximum width of the lower section, or between 15mm to 20mm less than the maximum width of the lower section. The minimum width of the waisted section may be between 10mm to 35mm less than the maximum width of the upper section, or between 15mm to 30mm less than the maximum width of the upper section, or between 20mm to 25mm less than the maximum width of the upper section, for example.

The minimum width of the waisted section may be between 75% to 95% of the maximum width of the lower section, or between 80% to 90% of the maximum width of the lower section, or between 83% to 88% of the maximum width of the lower section.

The minimum width of the waisted section may be between 73% to 92% of the maximum width of the upper section, or between 75% to 85% of the maximum width of the upper section, or between 80% to 85% of the maximum width of the upper section.In embodiments wherein the pouch is a closed pouch, the pouch may have a length of between 200mm to 240mm, or between 205mm to 235mm, or between 208mm to 230mm, for example. In embodiments wherein the pouch is an open pouch, the pouch may have a length of between 250mm to 300mm, or between 260mm to 290mm, or between 270mm to 280mm, for example. In embodiments wherein the pouch comprises a deployable drain, the pouch may have a length of between 150mm to 300mm when the deployable drain is in a deployed position, and a length of between 200mm to 240mm with the deployable drain in a stowed position.

Opposing edges of the waisted section may be concavely-curved. Opposing edges of the waisted section may have a radius of curvature, or a blend of radii of curvature, wherein the or each radii of curvature may be between 20mm to 60mm, or between 30mm to 50mm, or between 35mm to 45mm, for example. The or each radii of curvature may be approximately 40mm. Opposing edges of the waisted section may be configured in substantially the same way or form, and may be mirror images of each other, for example.

The upper and/or lower sections may be generally rounded in shape. For example, the upper section may comprise a continuously curved edge that extends from a first edge (e.g. a left-hand edge) of the waisted section to a second edge (e.g. a right-hand edge) of the waisted section. The lower section may comprise a continuously curved edge that extends from a first edge (e.g. a left-hand edge) of the waisted section to a second edge (e.g. a right-hand edge) of the waisted section.

The continuously curved edge of the upper and/or lower sections may be convexly curved. The continuously curved edge of the upper and/or lower sections may be absent any points of inflection or abrupt changes in contour. The continuously curved edge of the upper and/or lower sections may have a radius of curvature, or a blend of radii of curvature, wherein the or each radii of curvature may be between 40mm to 80mm, or between 55mm to 75mm, or between 60mm to 73mm, or between 65mm to 70mm, for example.

In embodiments, the upper and/or lower section may be elongated, and may comprise both a rounded portion and a generally rectangular section. The generally rectangular portion may comprise opposing straight edges. The rounded portion may be provided at a position distal from the waisted section. The generally rectangular portion may be provided at a position proximal to the waisted section.

A junction between the upper and/or lower sections and the waisted section may be demarcated by a single point of inflection between a left-hand edge of the upper or lower section and a left-hand edge of the waisted section, and by a single point of inflection between a right-hand edge of the upper or lower section and a right-hand edge of the waisted section.

A location of the minimum width of the waisted section may be at a distance of between 90mm to 125mm, or between 95mm to 120mm, or between 100mm to 115mm, or between 105mm to 115mm, or approximately 99mm, or 109mm, or 119mm from an uppermost edge of the ostomy pouch. A location of the minimum width of the waisted section may be at a distance of between 90mm to 125mm, or between 95mm to 120mm, or between 100mm to 115mm from a lowermost edge of the ostomy pouch. The distance may be between 45% to 60% of the length of the pouch, or between 47% to 57% of the length of the pouch, or between 50% to 55% of the length of the pouch.

The inner wall and the outer wall may be joined together by a single continuous edge seal. For example, where the pouch comprises a closed pouch, the single continuous edge seal may form a closed peripheral seal. Where the ostomy pouch comprises an open pouch, the single continuous edge seal may extend from a first edge (e.g. a left-hand edge) of a drain aperture to a second edge (e.g. a right-hand edge) of a drain aperture of the pouch. The single continuous edge seal may comprise a weld, which may optionally have a width of between 2mm to 6mm, or between 3mm to 5mm, or approximately 4mm, for example. The single continuous edge seal may have a constant width around the periphery of the ostomy pouch.

The inner wall and the outer wall may be formed from flexible sheet material. The flexible sheet material may comprise a single layer or a laminate of a plurality of layers. The flexible sheet material of the inner wall and/or the outer wall may comprise polyvinylidene chloride (PVDC) and/or ethylene-vinyl acetate (EVA). The inner wall and/or the outer wall may have a thickness of between 50 to 150 micrometres, or between 75 to 125 micrometres, or between 75 to 100 micrometres, for example.

The pouch may further comprise at least one comfort layer overlying at least a portion of one of the inner wall and outer walls. The pouch may comprise an inner comfort layer and/or an outer comfort layer.

Where present, the outer comfort layer may comprise a first part and a second part which may be joined to the outer wall so that the first part partially overlaps the second part in an overlap region. The first part and the second part may be separable from each other in the overlap region to form a window opening for viewing the cavity. The overlap region may be angled to extend horizontally when the pouch is in use. The first part and the second part of the outer comfort layer may be configured to slide over each other in the overlap region to accommodate expansion of the underlying outer wall. The first part and the second part may be joined to each other at a first end and at a second end of the overlap region. The first part and the second part may be welded to each other at the first end and at the second end of the overlap region, optionally as part of a peripheral weld of the pouch. External edges of the one or more parts of the outer comfort layer are shaped and sized to correspond to the shape, form and contours of the outer wall.

Where present, the inner comfort layer may comprise a single part or multiple parts. The inner comfort layer may cover only a portion of the inner wall. However, preferably the inner comfort layer covers substantially the whole of the inner wall. An aperture may be provided in the inner comfort layer corresponding to the location of the inlet in the inner wall of the pouch. The inner comfort layer may be shaped and sized to correspond to the shape, form and contours of the inner wall. The inner comfort layer may be provided with a wafer aperture that corresponds to the location of the inlet of the inner wall to permit fluid connection of the inlet of the inner wall to an ostomy wafer.

The at least one comfort layer may be formed from a flexible sheet material. The material of the flexible sheet material may comprise one or more of polyester, nylon, viscose, polyurethane, polyethylene, polypropylene, polyvinylidene chloride (PVDC) and ethylene-vinyl acetate (EVA). The at least one comfort layer may comprise a laminate of two or more layers. The at least one comfort layer may comprise at least one fabric layer and at least one film layer. The at least one film layer may be laminated to the at least one fabric layer, and optionally may be laminated to the at least one fabric layer over an entire area of the at least one comfort layer. The at least one fabric layer may comprise a woven or a non-woven textile layer. The fabric layer may comprise polyester, nylon, viscose, polyethylene or polypropylene. The at least one film layer may comprise polyurethane, polyvinylidene chloride (PVDC) or ethylene-vinyl acetate (EVA). The at least one comfort layer may have a thickness of between 50 to 1000 micrometres, or between 60 to 500 micrometres, or between 75 to 300 micrometres, or between 100 to 200 micrometres, for example.

The inner wall and the inner comfort layer may be joined together around their peripheral edges and/or the outer wall and the outer comfort layer may be joined together around their peripheral edges. The joining may be by use of welding, adhesive or equivalent means. A single joining operation may be used to join the inner comfort layer, the inner wall, the outer wall and the outer comfort layer together. For example a single weld may be used to join the four layers.

The pouch may comprise either an ostomy wafer that is located within or otherwise associated with the inlet of the inner wall, or a releasable coupling that is located within or is otherwise associated with the inlet of the inner wall. In embodiments, the releasable coupling may be configured for coupling with a body fitment component comprising an ostomy wafer. Where present, the ostomy wafer may extend through an aperture of the inner wall and/or inner comfort layer. The ostomy wafer may be provided with a releasable liner which may be removed by a user prior to securing the pouch to the ostomate, in use.

The pouch may comprise one or more further sections, for example a third section, and/or a fourth section. The one or more further sections may be separated from one or more other sections of the pouch by respective waisted sections. The waisted sections may have a minimum width which is less than the maximum width of the further section(s).

### Detailed Description of the Invention

In order that the invention may be more clearly understood one or more embodiments thereof will now be described, by way of example only, with reference to the accompanying drawings, of which:
Figure 1A is a side perspective view of an ostomy pouch not in accordance with the invention;
Figure 1B is a further side perspective view of the ostomy pouch of Figure 1A;
Figure 2A is an exploded perspective view of the ostomy pouch of Figures 1A and 1B;
Figure 2B is an exploded perspective view of the ostomy pouch of the invention.
Figure 3A is a side perspective view of an ostomy pouch;
Figure 3B is a further side perspective view of the ostomy pouch of Figure 2A;
Figure 4 is a side perspective view of an ostomy pouch;
Figure 5 is a side perspective view of an ostomy pouch not according to the present invention;
Figure 6 is an exploded perspective view of the ostomy pouch shown in Figure 5;
Figure 7 is a side cross-sectional view of the ostomy pouch of Figure 4;

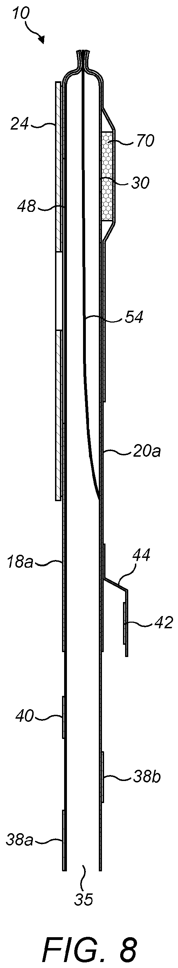

Figure 8 is a side cross-sectional view of the ostomy pouch of Figures 1A - 2B;
Figure 9 is a side cross-sectional view of part of a filter system for use with the present invention;
Figure 10 is a side perspective view of a filter cap for use with the present invention;
Figure 11 is a side perspective view of an ostomy pouch; and
Figure 12 is a side perspective view of an ostomy pouch.

When used herein and throughout the specification, the term "stomal output" refers to any gases, liquids or solids produced by an ostomate that may be secreted from a stoma or that exit a stoma of the ostomate. The stomal output may comprise gaseous, fluid, liquid and/or solid stomal output.

The term "stoma" refers to an opening in the body. Generally the stoma is a surgical opening in the torso of the body. In some instances, the term "stoma" also refers to internal tissue, organs or portions thereof that are exposed by the opening. By way of non-limiting example, internal tissue may be selected from colon, ileum, small intestine, large intestine, jejunum, and duodenum, and combinations thereof. The internal tissue may be an end or a loop of a small or large intestine.

The term "ostomate" refers to a subject that may have use of the ostomy pouch described herein. While ostomate usually refers to a subject with a surgical opening, as used herein, "ostomate" may refer to a subject who has a stoma, regardless of whether the stoma was created by surgery or other means.

The term "user" may refer to an ostomate, or to another person assisting the ostomate, for example, with emptying of the stomal output from the cavity.

Ostomy pouches disclosed herein may, for example, be used for managing a stoma created by an esophagostomy, a gastrostomy, a cholecystostomy, a choledochostomy, a cecostomy, a colostomy, a duodenostomy, an ileostomy, a jejunostomy, an appendicostomy, a tracheostomy, a urostomy, a nephrostomy, a ureterostomy, or a vesicostomy. The ostomy pouches disclosed herein may be used with additional devices including, but not limited to, a shunt, a catheter, a plug or a faecal management system.

In this specification locations and orientations of features may be described with reference to the ostomy pouch being "in use", "orientated as it would be in use" or similar. Such terms refer to the intended orientation of the ostomy pouch when it is adhered or otherwise secured to a body of an ostomate, e.g. with the ostomate in a standing position, irrespective of whether the ostomy pouch is currently performing such a use or the actual position of the ostomate. The terms "upper" and "lower" and related terms refer to the relative position of a part or portion of the ostomy pouch when orientated as it may be in use. For example, a section of the ostomy pouch may be referred to as an "upper" section of the ostomy pouch. In such an example, said section will be intended to be the uppermost section (in the vertical direction) of the ostomy pouch when attached to the body of a standing ostomate. However the reader skilled in the art will appreciate that before attachment to the ostomate said section may not always be the uppermost section and in addition when attached the section may not always be the uppermost section if the ostomate adopts a non-standing position, for example lying down.

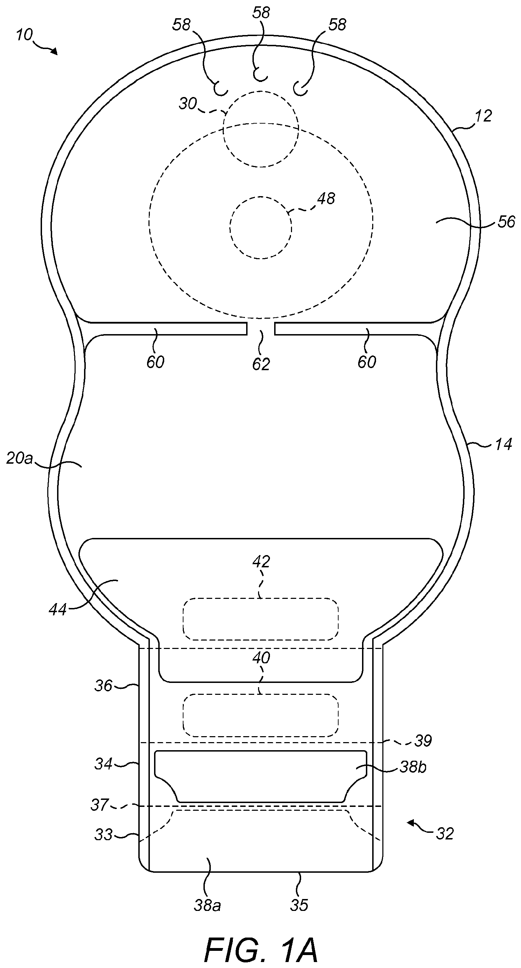

The terms "left-hand" and "right-hand" and related terms may refer to the ostomy pouch when viewed from the rear (for example, as shown in Figure 1A). Thus, as an illustrative example, a "left-hand" edge of the ostomy appliance will be towards a left-hand side of the ostomate in the situation where the ostomy pouch is attached to the front torso of the ostomate.

The terms "concave" and "convex" and related terms refer to shaping of features of the ostomy pouch when viewed from an exterior of the ostomy pouch. Thus, as an illustrative example, an ostomy wafer of circular shape would be considered to have a convexly shaped peripheral edge.

The terms "inner" and "outer" refer to the relative position of a part or portion of the ostomy pouch with reference to the body of an ostomate when the ostomy pouch is attached (e.g. adhesively or otherwise) to the body of the ostomate. "Inner" refers to a position relatively closer to the body of the ostomate than a comparative position that is "outer". "Outer" refers to a position relatively further away from the body of the ostomate than a comparative position that is "inner".

Ostomy pouches are commonly attached to the body of an ostomate by means of an ostomy wafer which includes an adhesive layer or layers. The ostomy wafer typically has an opening for the stoma sometimes referred to as a starter hole which may be cut to a required size by a user before attachment. The ostomy wafer typically comprises an adhesive layer on a body-facing side for adhering the ostomy wafer to the body of the ostomate. Typically, a release liner covers a body-facing side of the ostomy wafer that is removed by the user prior to fitting to the skin. In this specification, the term "ostomy wafer" may be used interchangeably with the terms "adapter," "wafer," "baseplate", or "layered adhesive wafer." In this specification, the term "ostomy wafer" includes ostomy wafers for a "two-piece appliance" and for a "one-piece appliance".

A "two-piece pouch" refers to an ostomy pouch where the ostomy wafer forms part of a separate body fitment component that is attached by a releasable coupling to a pouch. A two-piece pouch permits the body fitment component to be separated from the pouch without damage, so that at least one of the parts continues to be functionally usable. For example, the body fitment component may remain in place on the body of the ostomate. In contrast, a "one-piece pouch" refers to an ostomy pouch where the ostomy wafer is permanently attached to the appliance, to the extent that the ostomy wafer cannot easily be separated without risk of damaging the appliance. A one-piece pouch is intended to be used as an integral unit.

Ostomy pouches may commonly be configured as "closed" pouches or "open" pouches. In this specification a "closed pouch" refers to an ostomy pouch where it is not intended that stomal output is drained from the cavity. Thus, a closed pouch may typically be configured as a one-use, disposable and non-reusable pouch. In this specification an "open pouch" refers to an ostomy pouch where it is possible for the stomal output to be drained from the cavity and the pouch reused. Thus, an open pouch may be configured as a reusable pouch, such that it can be reused and emptied multiple times whilst attached to the body, although this is not essential. In an open pouch the stomal output may be drained intermittently as instigated by an action of the ostomate or may be drained intermittently or continuously due to the cavity being fluidly connected to a drain, for example a night drain line.

The use of a closed pouch or an open pouch may be, in part, due to user preference, but equally either a closed or open pouch may be more suited depending on the particular ostomate's needs, and depending on the position of the stoma for the ostomate. For example, for stomas formed via ileostomy the stomal output may tend to be looser and be easily drainable which may lead to an open pouch being suitable. For stomas formed by colostomy, the stomal output may tend to be more solid and may not be readily drained by a user. In such instances, a closed pouch may be more suited.

Conditional language, such as "can," "could," "might," or "may," unless specifically stated otherwise, or otherwise understood within the context as used, is generally intended to convey that certain embodiments include, while other embodiments do not include, certain features, elements, and/or steps. Thus, such conditional language is not generally intended to imply that features, elements, and/or steps are in any way required for one or more embodiments or that one or more embodiments necessarily include logic for deciding, with or without user input or prompting, whether these features, elements, and/or steps are included or are to be performed in any particular embodiment.

The Figures illustrate a series of embodiments. Where equivalent components are present between embodiments, like reference numerals have been used.

The pouch 10 comprises an inner wall 18b and an outer wall 20b which are sealed about their periphery and define a cavity for containing a stomal output. The pouch 10 is an open pouch, with the seal provided as a single, continuous seal about the perimeter of the pouch 10, except at a drain aperture 35, described in detail hereinbelow. The cavity includes a first, upper section 12, a second, lower section 14 which are generally rounded in shape with convex curved edges. The upper and lower sections 12, 14 are separated by a waisted section 13 which is narrower in width than upper and lower sections 12, 14.

The inner and outer walls 18b, 20b are formed of a flexible, plastics sheet material. The pouch 10 also includes inner and outer comfort layers 18a, 20a which overlie respective inner and outer walls 18b, 20b. The comfort layers 18a, 20a are formed of a woven, fabric material and define an outer surface of the pouch 10. In the illustrated embodiment, a single joining operation is used to join the inner comfort layer 18a, the inner wall 18b, the outer wall 20b and the outer comfort layer 20a together, here through welding.

Here, the maximum width of the upper section 12 is greater than the maximum width of the lower section 14, and the minimum width of the waisted section 13 is less than the maximum width of the lower section 14. Specifically, the maximum width of the upper section 12 is approximately 142mm, the maximum width of the lower section is approximately 139mm, and the minimum width of the waisted section 13 is approximately 119mm.

In the illustrated embodiment, a single joining operation is used to join the inner comfort layer 18a, the inner wall 18b, the outer wall 20b and the outer comfort layer 20a together, here through welding.

The outer comfort layer includes first and second parts 20a', 20a" which are joined to the outer wall 20b so that the first part 20a' partially overlaps the second part 20a" in an overlap region. In use, the first part 20a' and the second part 20a" are separable from each other in the overlap region to form a window opening for viewing the cavity.

A filter system is provided which includes a vent 30 provided in the outer wall 20b for venting of gaseous stomal output from the cavity. This can advantageously maximise the capacity of the pouch 10 for receiving liquid and solid stomal output, and prevent "bloating" or "ballooning" of the pouch 10 which may adversely affect discreetness of the pouch 10 in use. The filter system is provided within the upper section 12 of the pouch.

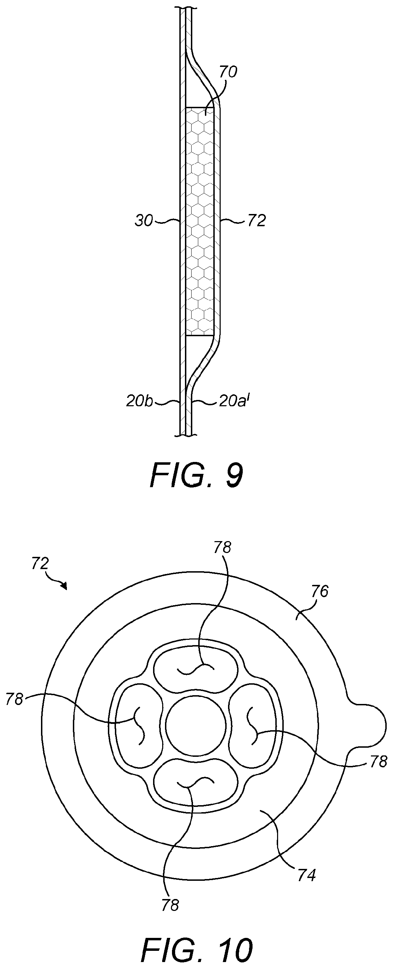

The vent 30 is provided with an odour filter 70 (e.g. a charcoal or activated carbon filter) for reducing the release of unpleasant odours from the cavity. Here, the odour filter 70 is substantially circular in shape, although other shapes and forms of odour filter are equally applicable, as will be appreciated. As described in detail below with reference to Figure 9 specifically, the odour filter 70 is located on an outer surface of the outer wall 20b, proximal to the position of the vent 30 such that gaseous stomal output release through the vent 30 is released from the pouch 10 via the filter 70 and subsequently through the gap between the first and second parts 20a', 20a" of the outer comfort layer 20a.

The filter system additionally includes a pre-filter arrangement comprising a protective panel 54 which defines a filter chamber 56 about the vent 30, and into which stomal output may enter, in use. An inlet is provided in the form of a plurality of c-cut apertures 58 in the protective panel 54. The apertures 58 allow gaseous stomal output to migrate into the filter chamber 56. The apertures 58 are positioned above and about the vent 30, specifically at a radial distance from the vent 30. The apertures 58 are also angled at approximately 45° with respect to a horizontal axis through the pouch 10. An outlet is provided to allow liquid stomal output to migrate out from the filter chamber 56 into the remainder of the cavity. The outlet is formed by a pair of bar welds 60 sealing the protective panel 54 to the outer wall 20b of the pouch 10, with a gap 62 therebetween.

The inner wall 18b comprises an opening therein defining a stomal inlet 48 in the pouch 10 for receiving stomal output into the cavity. Here, the stomal inlet 48 is provided within the upper section 12 of the pouch 10. An aperture 46 is provided in the inner comfort layer 18a defining a wafer aperture into which an ostomy wafer 24 is located and positioned over the stomal inlet 48, in use.

The ostomy wafer 24 includes a central aperture 22, an adhesive region 28, and a removable release liner 25 for exposing the adhesive region 28 which may subsequently be used to secure the pouch 10 to and about the stoma of the ostomate, in use. A tab 26 is provided on the release liner 25 to assist the user in removing the release liner 25. The ostomy wafer 24 is suitably secured to the inner wall 18b, e.g. through use of a further adhesive region. The adhesive region 28 is mouldable to the extent that it may be manipulated to adjust the shape and size the central aperture 22 according to the size and shape of the ostomate's stoma. When used here and throughout the specification, the term "mouldable" is intended to cover a component, here the adhesive region 28, which can be shaped under application of a force (e.g. rolling) by a user. In an alternative arrangement the adhesive region may be configured such that it may be shaped to fit the stoma by a user cutting the region to make the central aperture 22 the required size and shape, for example.

Pouch 10 is an "open" pouch 10 which includes a drain 32 for draining stomal output from the cavity. As shown, the drain 32 is formed in the lower section 14 of the pouch 10, and comprises a drain aperture 35 comprising an unsealed portion of the periphery of the pouch 10, i.e. a region of the periphery of the inner and outer walls 18b, 20b which is not sealed together. Stomal output may be released from the cavity through the drain aperture 35, in use.

The drain 32 is integral with the inner and outer walls 18b, 20b, and forms an elongated portion of the pouch 10 which extends downwardly from a lower edge of the lower section 14. The inner and outer comfort layers 18a, 20a are not provided over the drain 32. Similarly, the separation filter 50 aligns with the inner and outer comfort layers 18a, 20a does not extend into the drain 32. The separation filter here is not sealed to the inner and outer walls 18b, 20b at its lowermost edge, allowing for stomal output to drain from both the first and second cavity chambers of the cavity through the drain 32. In an alternative arrangement the lowermost edge of the separation filter 50 may extend at least partly into the drain 32. In this way, the lowermost edge may be folded with the drain (in the manner described below) thereby preventing output passing from one side of the pouch 10 to the other "under" the separation filter 50 with the drain 32 stowed.

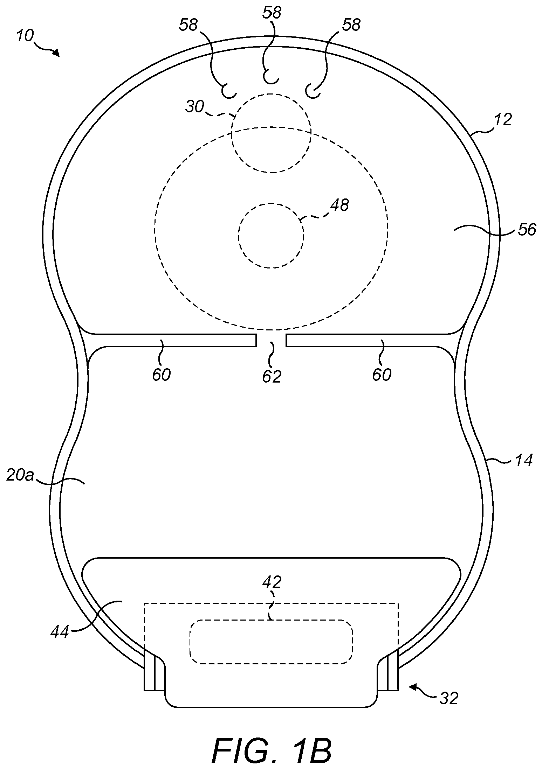

The drain 32 is a deployable drain 32 which is moveable between a deployed position (shown in Figure 1A) to a stowed position (shown in Figure 1B). Moving the drain 32 to the stowed position effectively closes off the drain aperture 35 preventing release of stomal output from the cavity. Specifically, the drain 32 is foldable between the deployed and stowed positions roughly about fold lines 37, 39 in the drain 32. The fold lines define the drain 32 into first, second and third segments 33, 34, 36, with the segments 33, 34, 36 being foldable on top of one another, e.g. about fold lines 37, 39 when moving from the deployed position to the stowed position.

Pursing strips 38a, 38b are provided on opposing sides of the drain 32, with a first pursing strip 38a provided on the portion of the inner wall 18b defining the first segment 33 of the drain 32, and a second pursing strip 38b provided on the portion of the outer wall 20b defining the second segment 34 of the drain 32. The pursing strips 38a, 38b provide localised rigidity to the drain 32 and assist with the opening of the drain aperture 35, in use. Specifically, the pursing strips 38a, 38b may be squeezed laterally to arch the pursing strips 38a, 38b (and hence the inner and outer walls 18b, 20b respectively) in opposing directions to open the drain aperture 35.

The drain 32 includes a first fastening element in the form of a strip of hook fasteners 40 provided on an outer surface of the inner wall 18b. Specifically, the strip of hook fasteners 40 is provided on the outer surface of the portion of the inner wall 18b defining the third segment 36 of the drain 32. A corresponding second fastening element 42 is provided on a flap 44 attached (e.g. adhesively secured) to an outer surface of the outer comfort layer 20a. The second fastening element similarly takes the form of a strip of hook fasteners 42.

In use, the drain 32 is moved from the deployed position to the stowed position by folding the drain 32 about fold lines 37, 39 upwards and away from the ostomate, by first bringing the first segment 33 adjacent to and overlying the second segment by folding along fold line 37, and subsequently folding the folded first and second segments 33, 34 about the fold line 39 bringing those segments adjacent to and overlying the third segment 36. Finally, the drain 32 is folded once more bringing the folded drain 32 to a position where it overlies a portion of the outer comfort layer 20a as shown in Figure 1B, with the flap 44 folded downwards bringing the second fastening element 42 into contact and engagement with the first fastening element 40 to retain the drain 32 in the stowed position. The drain 32 is moved from the stowed position to the deployed position in the opposite manner.

The pouch 10 has a length of approximately 208mm with the drain 32 stowed, and a length of approximately 270mm with the drain 32 deployed.

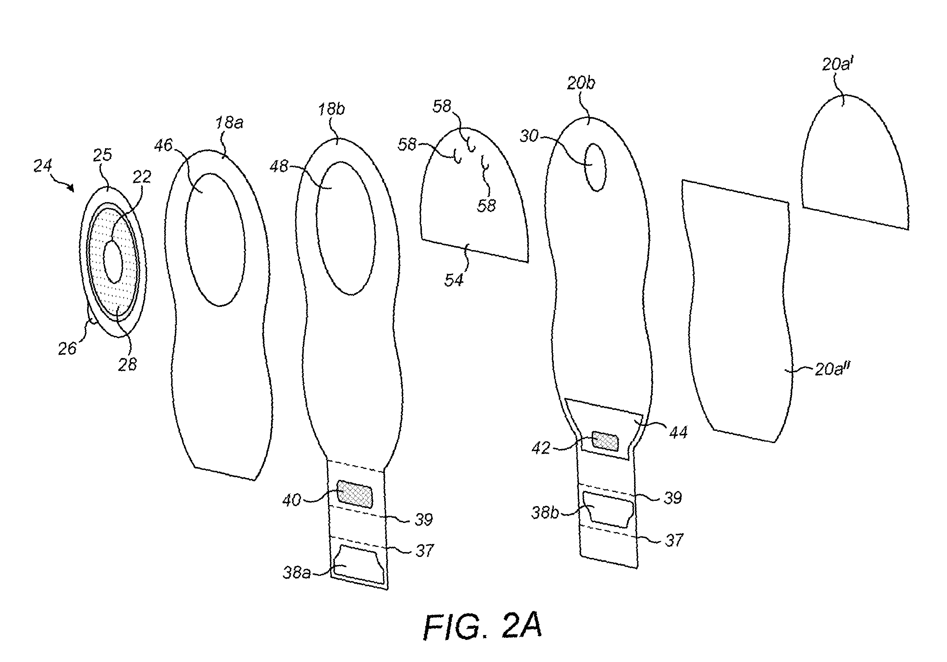

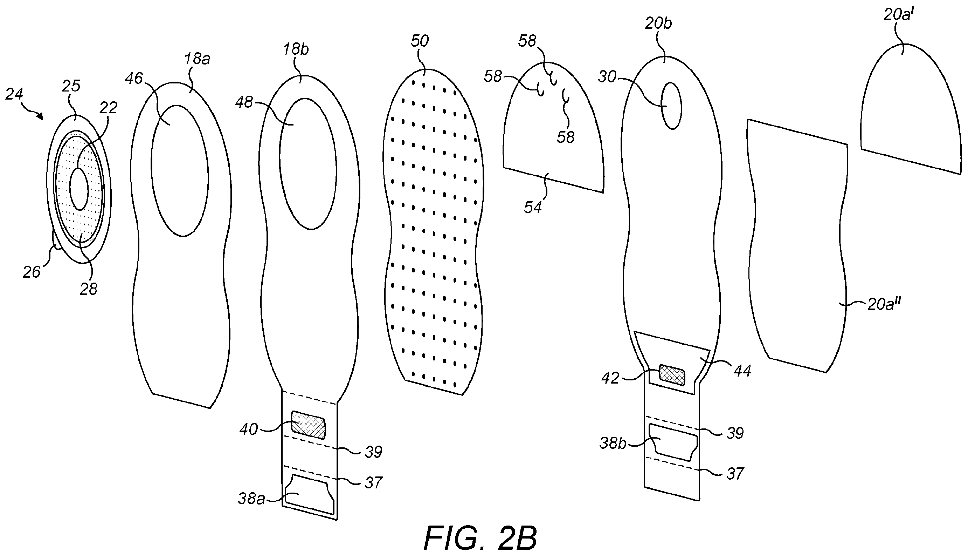

The pouch 10 of the invention is shown in Figure 2B, which includes a separation filter 50 provided within the pouch 10, positioned between the inner and outer walls 18b, 20b, and separating the cavity into two cavity chambers. The separation filter 50 is fluid-permeable, and is operable to filter fluid (i.e. gaseous and liquid) stomal output from solid stomal output. Specifically, and in use, stomal output is received through the stomal inlet 48 into the first chamber of the cavity. This stomal output may be both fluid and solid. The separation filter 50 is arranged to allow passage of fluid stomal output into the second chamber, proximal to the outer wall 20b, whilst retaining the solid stomal output in the first chamber, proximal to the inner wall 18b. In the illustrated embodiment, the separation filter 50 is shaped and sized substantially the same as the inner and outer walls 18b, 20b, and is sealed about its periphery (except in a region proximal to the drain 32) to the inner and outer walls 18b, 20b.

The separation filter 50 in combination with the pre-filter arrangement advantageously prevents solid stomal output, and substantially all of the liquid stomal output from coming into contact with the vent 30, thereby preventing or reducing the likelihood of the vent 30 becoming clogged and being unable to adequately vent the cavity.

Figures 9 and 10 further illustrate the odour filter 70 and its incorporation into pouch 10, and also a filter cap 72 for use with the filter 70.

As shown in Figure 9, and described hereinabove, the odour filter 70 is substantially circular in shape, and is located on an outer surface of the outer wall 20b, proximal to the position of the vent 30 such that gaseous stomal output release through the vent 30 is released from the pouch 10 via the filter 70 and subsequently through the gap between the first and second parts 20a', 20a" of the outer comfort layer 20a. A membrane (not shown) is provided about the filter 70, and specifically about the portion of the filter 70 exposed to the interior of the pouch 10 through vent 30. The membrane is typically perforated and acts as a further barrier to fluid and possibly solid stomal output from coming into contact with the filter 70 itself. The membrane is advantageously provided with a hydrophobic coating, further preventing liquid and/or solid stomal output from adhering to the membrane and preventing adequate airflow through the filter 70.

A filter cap 72 is provided on the outer surface of the filter 70 and acts to protect the filter 70 from damage. The filter cap includes a central region 74 shaped so as to fit over the filter 70 and a peripheral region 76 which serves as a portion for adhering the filter cap 72 to the outer wall 20b of the pouch 10. A series of s-slits 78 are provided in the central region 74 allowing for venting of gas from the filter 70 and out through the outer comfort layer 20a.

Adhesive layers (not shown) are provided either side of the filter 70 for adhering the filter 70 to the outer wall 20b on a first side and to the filter cap 72 on the opposing side.

For open pouches, such as pouch 10, an ostomate may use the same pouch for a prolonged period of time. Accordingly, having the filter 70 external to the main pouch cavity and minimising exposure of the filter 70 to liquid and solid stomal output even with such extended use advantageously reduces the likelihood of the filter system clogging and ultimately the pouch 10 ballooning.

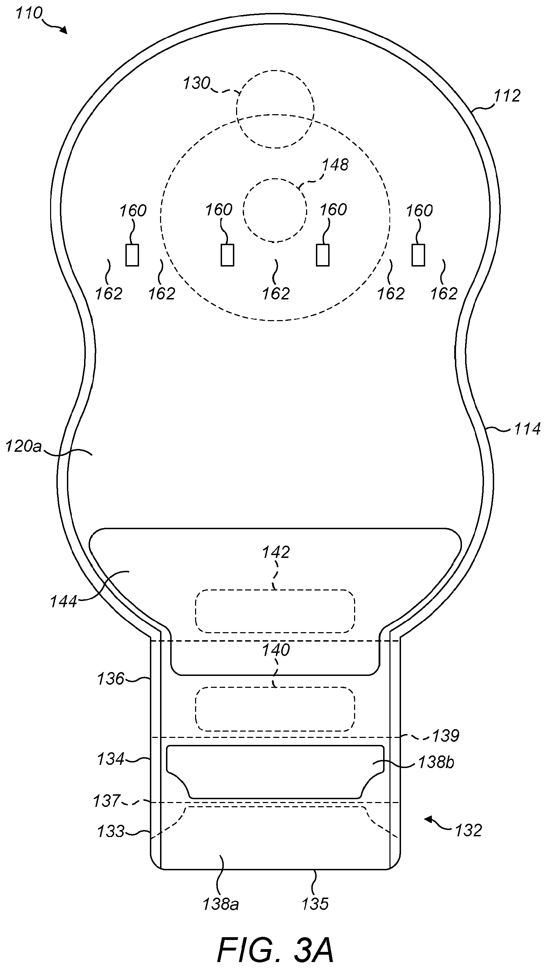

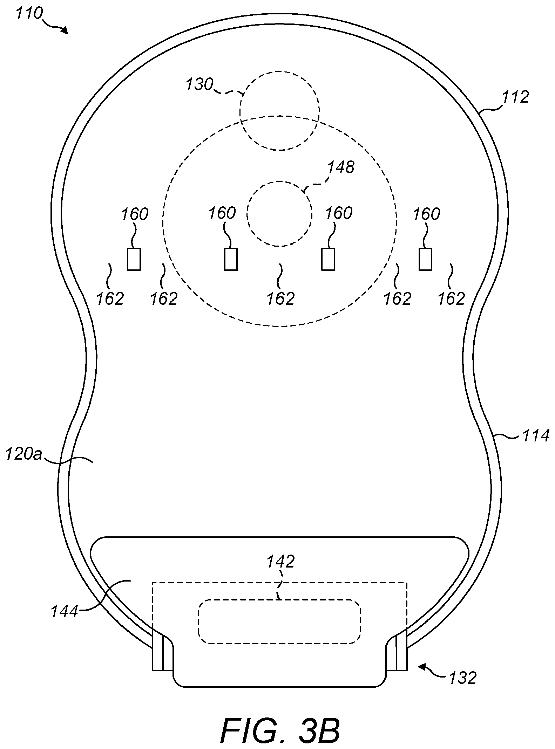

Figures 3A and 3B illustrate a pouch 110.

Where the features of pouch 110 are equivalent to those of pouch 10, like reference numerals have been used. Equivalent features are configured in the same way in pouch 110 as pouch 10 described herein, unless otherwise stated herein. For features that are common, reference should be made to the preceding description also.

Pouch 110 is formed of an inner wall 118b and an outer wall 120b sealed about their periphery and define a cavity for containing a stomal output. Again, the cavity includes a first, upper section 112, and a second, lower section 114 which are generally rounded in shape with convex curved edges. The upper and lower sections 112, 114 are separated by a waisted section 113 which is narrower in width than upper and lower sections 112, 114. The pouch 110 is dimensioned approximately equal to the dimensions of pouch 10 discussed herein.

As with pouch 10, the inner and outer walls 118b, 120b are formed of a flexible, plastics sheet material and are provided with inner and outer comfort layers 118a, 120a formed of woven, fabric material and which overlie respective inner and outer walls 118b, 120b. Again, a stomal inlet 148 is provided in the upper section 112 of the pouch 110 for receiving stomal output into the cavity, with an ostomy wafer 124 located within an aperture 146 in the inner comfort layer 118a and positioned over the stomal inlet 148, in use. The ostomy wafer 124 is configured in the same way as ostomy wafer 24 de- scribed hereinabove. The pouch of the invention includes a separation filter (as with the pouch shown in Figure 2B) and a deployable drain 132 configured in substantially the same manner as separation filter 50 and drain 32 described herein. As with pouch 10, the deployable drain 132 is moveable between a deployed position (Figure 3A) and a stowed position (Figure 3B) in the same manner, by folding the drain 132 about various fold lines 137, 139 and securing in position using fasteners 140, 142.

Pouch 110 differs from pouch 10 in the formation of the filter system.

Pouch 110 again includes a vent 130 provided in the outer wall 120b of the pouch 110 for venting of gaseous stomal output from the cavity. As with pouch 10, the vent 130 is provided with an odour filter (e.g. a charcoal or activated carbon filter - not shown) located on an outer surface of the outer wall 20b, proximal to the position of the vent 130, and for reducing the release of unpleasant odours from the cavity. The filter may be configured in the same way as filter 70 described hereinabove. The filter system is similarly provided within the upper section 112 of the pouch.

The pre-filter arrangement of pouch 110 includes a protective panel 154 defining a filter chamber 156 about the vent 130, and into which stomal output may enter, in use. The protective panel 154 is sealed to the outer wall a plurality of attachment regions formed by spot welds 160 across the width of the pouch 110. The spot welds 160 have gaps 162 therebetween (or between a spot weld 160 and the sealed edge of the pouch 110) together forming an outlet allowing for liquid stomal output to migrate out from the filter chamber 156 into the remainder of the cavity. Here, the outlet also forms an inlet for allowing gaseous and liquid stomal output to migrate into the filter chamber 156.

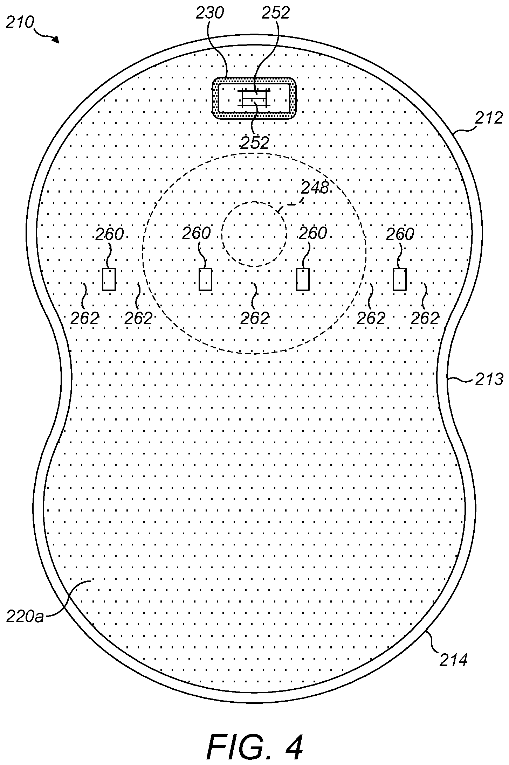

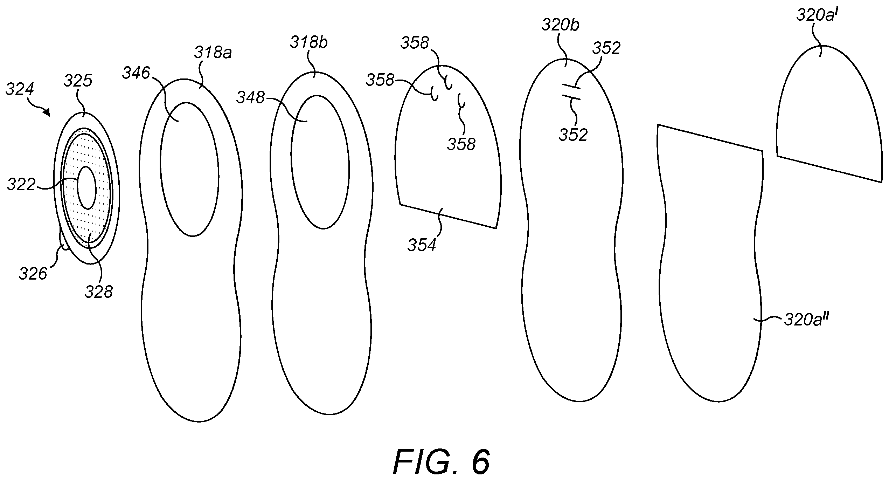

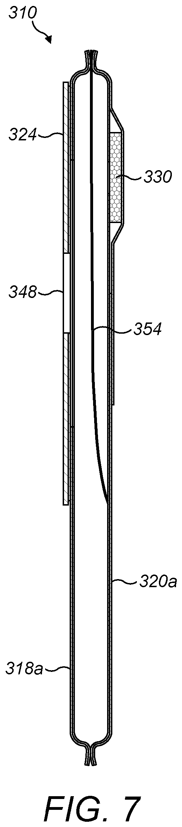

Figures 4-7 illustrate further ostomy pouches 210, 310.

Where the features of pouches 210, 310 are equivalent to those of pouches 10, 110 described above, like reference numerals have been used. Equivalent features are configured in the same way in pouches 210, 310 as pouch 10, 110 described herein, unless otherwise stated herein. For features that are common, reference should be made to the preceding description also.

Figure 4 illustrates an ostomy pouch 210. The pouch 210 is formed of an inner wall 218b and an outer wall 220b formed of flexible, plastics sheet material sealed about their periphery and define a cavity for containing a stomal output, with inner and outer comfort layers 218a, 220a provided. Again, the cavity includes a first, upper section 212, and a second, lower section 214 which are generally rounded in shape with convex curved edges. The upper and lower sections 212, 214 are separated by a waisted section 213 which is narrower in width than upper and lower sections 212, 214. Again, a stomal inlet 248 is provided in the upper section 212 of the pouch 210 for receiving stomal output into the cavity. The pouch 210 includes a separation filter (as with the pouch 10 shown in Figure 2B) and a filter system configured in a similar manner as the filter system of pouch 110-see below.

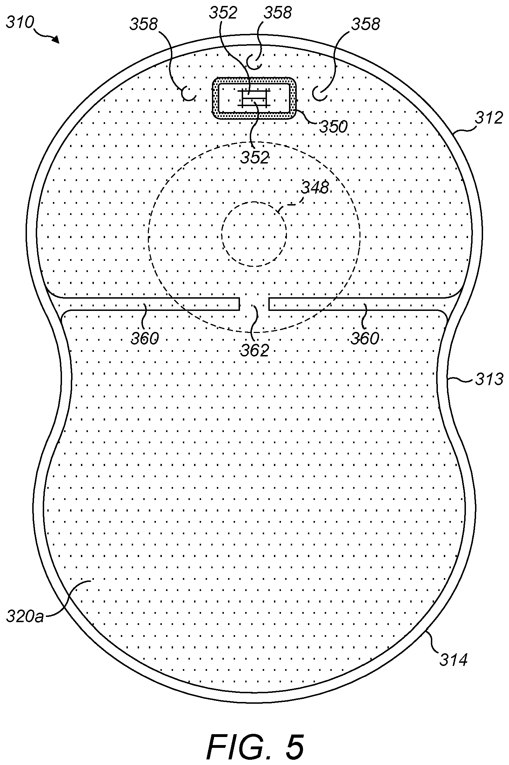

Figures 5 - 7 illustrate an ostomy pouch 310. The pouch 310 is 15 formed of an inner wall 318b and an outer wall 320b formed of flexible, plastics sheet material sealed about their periphery and define a cavity for containing a stomal output, with inner and outer comfort layers 318a, 320a provided. Again, the cavity includes a first, upper section 312, and a second, lower section 314 which are generally rounded in shape with convex curved edges. The upper and lower sections 312, 314 are separated by a waisted section 313 which is narrower in width than upper and lower sections 312, 314. Again, a stomal inlet 348 is provided in the upper section 312 of the pouch 310 for receiving stomal output into the cavity. The pouch 310 may similarly include a separation filter (as with the variant of pouch 10 shown in Figure 2B) and a filter system configured in a similar manner as the filter system of pouch 10 - see below.

Pouches 210, 310 differ in that they are "closed" pouches which do not include a drain. Rather, the inner and outer walls 218b, 220b; 318b, 320b are sealed about their entire periphery and define a sealed cavity for containing a stomal output.

Pouches 210, 310 further differ in the configuration of the filter system, and specifically in the configuration of the odour filter forming part of the filter system of pouches 210, 310 compared with the filter 70 provided in pouches 10, 110. Specifically, the filter systems of pouches 210, 310 comprise a strip filter configured to be provided on the interior surface of the outer wall 220b, 320b, and hence being exposed to the interior of the pouch 210, 310. Specifically, the strip filters are configured to be secured to the interior surface of the outer walls 220b, 330b at respective weld regions 230, 330 securing the filters to the outer wall at or proximal to the periphery of the filters. Strip filters may be exposed at ends thereof, allowing gaseous stomal output to enter the filter through said open ends, and out through a gas permeable rear face of the filter. Slits 252, 352are provided in the outer wall 220b, 320b positioned adjacent to the rear face of the filter 230, 330 for venting of gas from within the pouch 210, 310 through the filter 230, 330 and out through the slits. The slits 252, 352 comprise a pair of slits arranged horizontally across the pouch for part of the width of the filter 230, 330.

Strip filters typically provide a cost-effective solution for providing an odour filter in the pouch 210, 310 when compared with filter 70 in the preceding embodiments, but with the downside that strip filters themselves are exposed directly to liquid and potentially solid stomal output within the pouch 210, 310 which may clog the pouch if exposed for a long period of time. For closed pouches, such as pouches 210, 310 which are designed for wear for a much shorter period of time when compared with open pouches (e.g. pouch 10, 110), the filter is exposed for a much shorter period of time and therefore less likely to clog before the end of its use period.

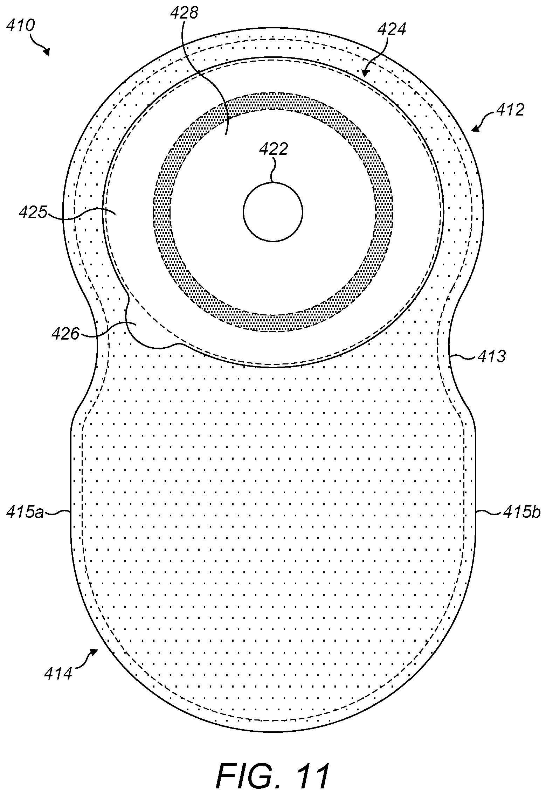

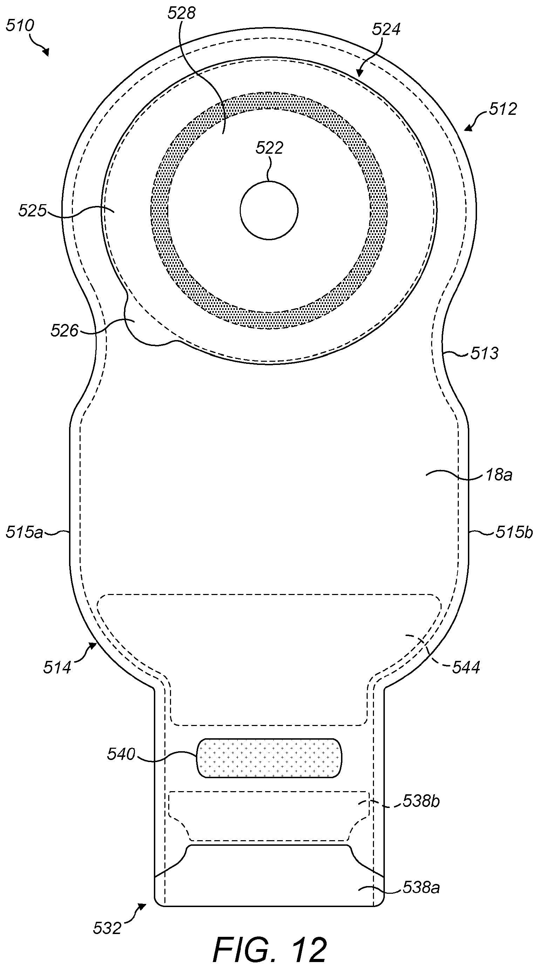

Figures 11 and 12 illustrate fifth and sixth ostomy pouches 410, 510.

Where the features of pouches 410, 510 are equivalent to those of pouches 10, 110, 210, 310 described above, like reference numerals have been used. Equivalent features are configured in the same way in pouches 410, 510 as pouches 10, 110, 210, 310 described herein, unless otherwise stated herein. For features that are common, reference should be made to the preceding description also.

Pouch 410 is configured substantially in the same way as pouch 210 shown in Figure 4, and is again a closed pouch which has inner and outer walls 418b, 420b sealed about their entire periphery and define a sealed cavity for containing a stomal output. Pouch 410 differs in that the lower section 414 of pouch 410 is shaped different, and in particular is more elongated. Here, the lower section 414 of the pouch 410 includes straight edge portions 415a, 415b extending from the waisted section 413 to define a lower section 414 which has a generally rectangular portion proximal to the waisted section 413, and a generally rounded section at a lowermost edge of the pouch 410. The elongated lower section 414 may advantageously have a greater capacity for storing stomal output therein.

Pouch 510 is configured substantially in the same way as pouch 110 shown in Figures 3A and 3B, and is again an open pouch which has inner and outer walls 518b, 520b sealed about their periphery and define a cavity for containing a stomal output with a drain 532 extending from the lowermost edge of the pouch 510. The drain 532 is configured in substantially the same manner as drain 132 of pouch 110. Pouch 510 differs in that the lower section 514 of pouch 510 is shaped different, and in particular is more elongated. As with pouch 410, the lower section 514 of the pouch 510 includes straight edge portions 515a, 515b extending from the waisted section 513 to define a lower section 514 which has a generally rectangular portion proximal to the waisted section 513, and a generally rounded section at a lowermost edge of the pouch 510.

The one or more embodiments are described above by way of example only. Many variations are possible without departing from the scope of protection afforded by the appended claims.

## Claims

1. An ostomy pouch (10, 110, 210, 310, 410, 510), comprising:
an inner wall (18b, 118b, 218b, 318b, 418b, 518b) and an outer wall (20b, 120b, 220b, 320b, 420b, 520b) sealed about at least part of the periphery thereof to define a cavity for containing a stomal output, the cavity including an upper section (12, 112, 212, 312, 412, 512), a lower section (14, 114, 214, 314, 41, 514) and a waisted section (13, 113, 213, 313, 413, 513) located between the upper section (12, 112, 212, 312, 412, 512) and the lower section (14, 114, 214, 314, 41, 514); and
a filter system, comprising:
a vent (30, 130, 230, 330) in the outer wall (20b, 120b, 220b, 320b, 420b, 520b) of the pouch for the release of gas from within the cavity to outside of the pouch; and
a pre-filter arrangement comprising a protective panel (54, 154, 354) which defines a filter chamber (56, 156) about the vent (30, 130, 230, 330), and into which stomal output may enter, in use,
**characterised in that** the filter chamber (56, 156) comprises an outlet (62, 162, 262, 362) allowing liquid stomal output to migrate out from the filter chamber (56, 156), and wherein the ostomy pouch further comprises a separation wall (50) between the inner (18b) and outer (20b) walls defining the cavity into first and second cavity chambers, wherein the separation wall (50) comprises a filtering element which is fluid-permeable operable to filter fluid stomal output from solid stomal output.

2. An ostomy pouch (10, 110, 210, 310, 410, 510) as claimed in claim 1, wherein the pre-filter arrangement is configured to control the content of stomal output in contact with the vent (30, 130, 230, 330).

3. An ostomy pouch (10, 110, 210, 310, 410, 510) as claimed in claim 1 or claim 2, wherein the filter chamber (56, 156) comprises an inlet allowing gaseous stomal output to migrate into the filter chamber.

4. An ostomy pouch (10, 310) as claimed in claim 3, wherein the inlet comprises one or more apertures (58, 358) in the protective panel (54, 354).

5. An ostomy pouch (10, 310) as claimed in claim 4, wherein the one or more apertures (58, 358) comprise a cut or slot in the protective panel (54, 354).

6. An ostomy pouch (10, 310) as claimed in claim 5, wherein the one or more apertures comprise a c-cut or an s-cut (58, 358) in the protective panel (54, 354).

7. An ostomy pouch (10, 310) as claimed in any of claims 3 to 6, wherein the inlet (58, 358) for the filter chamber acts as the outlet for liquid stomal output.

8. An ostomy pouch (10, 110, 210, 310, 410, 510) as claimed in any preceding claim, wherein the outlet (62, 162, 262, 362) is formed between the protective panel (54, 154, 354) and the outer wall (20b, 120b, 220b, 320b, 420b, 520b) of the pouch.

9. An ostomy pouch (10, 110, 210, 310) as claimed in claim 8, wherein the protective panel (54, 154, 354) is attached to the outer wall (20b, 120b, 220b, 320b) of the pouch at one or more attachment regions (60, 160, 260, 360), the one or more attachment regions thereby defining an outlet in the form of one or more gaps (62, 162, 262, 362) between adjacent attachment regions (60, 160, 260, 360), and/or between an attachment region (60, 160, 260, 360) and the peripheral seal forming the cavity.

10. An ostomy pouch (10, 110, 210, 310) as claimed in claim 9, wherein the protective panel (54, 154, 354) is attached to the outer wall (20b, 120b, 220b, 320b) of the pouch at the one or more attachment regions by one or more welds (60, 160, 260, 360).

11. An ostomy pouch (10, 110, 210) as claimed in claim 10, wherein the one or more welds (60, 160, 260) comprise a plurality of spot welds (60, 160, 260), the plurality of spot welds defining multiple attachment regions and multiple gaps (62, 162, 262) therebetween.

12. An ostomy pouch (310) as claimed in claim 10, wherein the one or more welds (360) comprise a pair of bar welds (360), the pair of bar welds defining a pair of attachment regions with a single gap (362) therebetween.

13. As ostomy pouch (10, 110, 510) as claimed in any preceding claim, comprising a drain (32, 132, 532) for releasing stomal output from the cavity.

## Patentansprüche

1. Ein Ostomiebeutel (10, 110, 210, 310, 410, 510), umfassend:
eine Innenwand (18b, 118b, 218b, 318b, 418b, 518b) und eine Außenwand (20b, 120b, 220b, 320b, 420b, 520b), die zumindest über einen Teil ihres Umfangs abgedichtet sind, um einen Hohlraum zur Aufnahme eines Stomaausgangs zu definieren, wobei der Hohlraum einen oberen Abschnitt (12, 112, 212, 312, 412, 512), einen unteren Abschnitt (14, 114, 214, 314, 41, 514) und einen taillierten Abschnitt (13, 113, 213, 313, 413, 513), der zwischen dem oberen Abschnitt (12, 112, 212, 312, 412, 512) und dem unteren Abschnitt (14, 114, 214, 314, 41, 514) angeordnet ist, umfasst; und
ein Filtersystem, umfassend:
eine Entlüftung (30, 130, 230, 330) in der Außenwand (20b, 120b, 220b, 320b, 420b, 520b) des Beutels für die Abgabe von Gas aus dem Hohlraum an die Außenseite des Beutels; und
eine Vorfilteranordnung, die eine Schutzplatte (54, 154, 354) umfasst, die eine Filterkammer (56, 156) um die Entlüftung (30, 130, 230, 330) herum bildet, und in die die Stomaausgänge im Gebrauch eindringen können,
**dadurch gekennzeichnet, dass** die Filterkammer (56, 156) einen Auslass (62, 162, 262, 362) umfasst, der es dem flüssigen Stomaausgang ermöglicht, aus der Filterkammer (56, 156) auszutreten, und wobei der Ostomiebeutel ferner eine Trennwand (50) zwischen der Innenwand (18b) und der Außenwand (20b) umfasst, die den Hohlraum in eine erste und eine zweite Hohlraumkammer unterteilt, wobei die Trennwand (50) ein Filterelement umfasst, das flüssigkeitsdurchlässig ist und dazu dient, den flüssigen Stomaausgang aus dem festen Stomaausgang zu filtern.

2. Ein Ostomiebeutel (10, 110, 210, 310, 410, 510) nach Anspruch 1, wobei die Vorfilteranordnung derart ausgebildet ist, dass sie den Inhalt des Stomaausgangs in Kontakt mit der Entlüftung (30, 130, 230, 330) kontrolliert.

3. Ein Ostomiebeutel (10, 110, 210, 310, 410, 510) nach Anspruch 1 oder Anspruch 2, wobei die Filterkammer (56, 156) einen Einlass umfasst, der es gasförmigen Stomaausgängen ermöglicht, in die Filterkammer auszutreten.

4. Ein Ostomiebeutel (10, 310) nach Anspruch 3, wobei der Einlass eine oder mehrere Öffnungen (58, 358) in der Schutzplatte (54, 354) umfasst.

5. Ein Ostomiebeutel (10, 310) nach Anspruch 4, wobei die eine oder die mehreren Öffnungen (58, 358) einen Schnitt oder Schlitz in der Schutzplatte (54, 354) umfassen.

6. Ein Ostomiebeutel (10, 310) nach Anspruch 5, wobei die eine oder die mehreren Öffnungen einen C-Schnitt oder einen S-Schnitt (58, 358) in der Schutzplatte (54, 354) umfassen.

7. Ein Ostomiebeutel (10, 310) nach einem der Ansprüche 3 bis 6, wobei der Einlass (58, 358) für die Filterkammer als Auslass für den flüssigen Stomaausgang dient.

8. Ein Ostomiebeutel (10, 110, 210, 310, 410, 510) nach einem der vorhergehenden Ansprüche, wobei der Auslass (62, 162, 262, 362) zwischen der Schutzplatte (54, 154, 354) und der Außenwand (20b, 120b, 220b, 320b, 420b, 520b) des Beutels ausgebildet ist.

9. Ein Ostomiebeutel (10, 110, 210, 310) nach Anspruch 8, wobei die Schutzplatte (54, 154, 354) an der Außenwand (20b, 120b, 220b, 320b) des Beutels an einem oder mehreren Befestigungsbereichen (60, 160, 260, 360) befestigt ist, wobei der eine oder die mehreren Befestigungsbereiche dadurch einen Auslass in Form eines oder mehrerer Lücken (62, 162, 262, 362) zwischen benachbarten Befestigungsbereichen (60, 160, 260, 360) und/oder zwischen einem Befestigungsbereich (60, 160, 260, 360) und der den Hohlraum bildenden Umfangsdichtung definieren.

10. Ein Ostomiebeutel (10, 110, 210, 310) nach Anspruch 9, wobei die Schutzplatte (54, 154, 354) an der Außenwand (20b, 120b, 220b, 320b) des Beutels an dem einen oder den mehreren Befestigungsbereichen durch eine oder mehrere Schweißnähte (60, 160, 260, 360) befestigt ist.

11. Ein Ostomiebeutel (10, 110, 210) nach Anspruch 10, wobei die eine oder mehreren Schweißnähte (60, 160, 260) eine Vielzahl von Schweißpunkten (60, 160, 260) umfassen, wobei die Vielzahl von Schweißpunkten mehrere Befestigungsbereiche und mehrere Lücken (62, 162, 262) dazwischen definieren.

12. Ein Ostomiebeutel (310) nach Anspruch 10, wobei die eine oder die mehreren Schweißnähte (360) ein Paar von Stangenschweißnähten (360) umfassen, wobei das Paar von Stangenschweißnähten ein Paar von Befestigungsbereichen mit einer einzigen Lücke (362) dazwischen definiert.

13. Ein Ostomiebeutel (10, 110, 510) nach einem der vorhergehenden Ansprüche, umfassend eine Drainage (32, 132, 532) zum Ablassen des Stomaausgangs aus dem Hohlraum.

## Revendications

1. Poche (10, 110, 210, 310, 410, 510) de stomie, comprenant :
une paroi (18b, 118b, 218b, 318b, 418b, 518b) intérieure et une paroi (20b, 120b, 220b, 320b, 420b, 520b) extérieure rendues étanches autour d'au moins une partie de leur périphérie pour définir une cavité contenant une sortie stomale, la cavité ayant une partie (12, 112, 212, 312, 412, 512) supérieure et une partie (14, 114, 214, 314, 41, 514) inférieure et une partie (13, 113, 213, 313, 413, 513) rétrécie, placée entre la partie (12, 112, 212, 312, 412, 512) supérieure et la partie (14, 114, 214, 314, 41, 514) inférieure ; et
un système de filtre comprenant :
un évent (30, 130, 230, 330) dans la paroi (20b, 120b, 220b, 320b, 420b, 520b) extérieure de la poche pour le dégagement de gaz de l'intérieur de la cavité à l'extérieur de la poche ; et
un agencement d'avant filtre comprenant un panneau (54, 154, 354) protecteur, qui définit une chambre (56, 156) de filtre autour de l'évent (30, 130, 230, 330), et dans lequel de la sortie stomale peut entrer en utilisation, **caractérisée en ce que** la chambre de filtration (56, 156) comprend une sortie (62, 162, 262, 362) permettant à la sortie stomale liquide de migrer hors de la chambre de filtre (56, 156), et dans laquelle la poche de stomie comprend en outre une paroi de séparation (50) entre les parois intérieure (18b) et extérieure (20b) définissant la cavité en des première et deuxième chambres, la paroi de séparation (50) comprenant un élément filtrant qui est perméable aux fluides et peut fonctionner pour séparer par filtration une sortie stomale fluide d'une sortie stomale solide.

2. Poche (10, 110, 210, 310, 410, 510) de stomie suivant la revendication 1, dans laquelle l'agencement d'avant filtre est configuré pour régler le contenu de la sortie stomale en contact avec l'évent (30, 130, 230, 330).

3. Poche (10, 110, 210, 310, 410, 510) de stomie suivant la revendication 1 ou la revendication 2, dans laquelle la chambre (56, 156) de filtre comprend une sortie permettant à de la sortie stomale gazeuse de migrer dans la chambre de filtre.

4. Poche (10, 310) de stomie suivant la revendication 3, dans laquelle l'entrée comprend une ou plusieurs ouvertures (58, 358) dans le panneau (54, 354) protecteur.

5. Poche (10, 310) de stomie suivant la revendication 4, dans laquelle la une ou les plusieurs ouvertures (58, 358) comprennent une découpe ou une fente dans le panneau (54, 354) protecteur.

6. Poche (10, 310) de stomie suivant la revendication 5, dans laquelle la une ou les plusieurs ouvertures (58, 358) comprennent une découpe en C ou une découpe en S dans le panneau (54, 354) protecteur.

7. Poche (10, 310) de stomie suivant l'une quelconque des revendications 3 à 6, dans laquelle l'entrée (58, 358) pour la chambre de filtre sert de sortie pour la sortie stomale de liquide.

8. Poche (10, 110, 210, 310, 410, 510) de stomie suivant l'une quelconque des revendications précédentes, dans laquelle la sortie (62, 162, 262, 362) est formée entre le panneau (54, 154, 354) protecteur et la paroi (20b, 120b, 220b, 320b, 420b, 520b) extérieure de la poche.

9. Poche (10, 110, 210, 310) de stomie suivant la revendication 8, dans laquelle le panneau (54, 154, 354) protecteur est fixé à la paroi (20b, 120b, 220b, 320b) extérieure de la poche en une ou en plusieurs régions (60, 160, 260, 360) de fixation, les unes ou plusieurs régions de fixation définissant ainsi une sortie sous la forme d'un ou de plusieurs intervalles (62, 162, 262, 362) entre des régions (60, 160, 260, 360) de fixation voisines, et/ou entre une région (60, 160, 260, 360) de fixation et le joint périphérique étanche formant la cavité.

10. Poche (10, 110, 210, 310) de stomie suivant la revendication 9, dans laquelle le panneau (54, 154, 354) protecteur est fixé à la paroi (20b, 120b, 220b, 320b) extérieure de la poche en une ou en plusieurs région de fixation par une ou plusieurs soudures (60, 160, 260, 360).

11. Poche (10, 110, 210) de stomie suivant la revendication 10, dans laquelle la une ou les plusieurs soudures (60, 160, 260) comprend une pluralité de soudures (60, 160, 260) par point, la pluralité de soudures par point définissant de multiples régions de fixation et de multiples intervalles (62, 162, 262) entre elles.

12. Poche (310) de stomie suivant la revendication 10, dans laquelle la une ou les plusieurs soudures (360) comprennent une paire de soudures (360) par barre, la paire de soudures par barre définissant une paire de régions de fixation avec un seul intervalle (362) entre elles.

13. Poche (10, 110, 510) de stomie suivant l'une quelconque des revendications précédentes, comprenant un drain (32, 132, 532) pour relâcher de la sortie stomale de la cavité.
